# EUROPEAN PATENT APPLICATION

(11) **EP 2 719 768 A1**
(43) Date of publication of application: **16.04.2014**
(21) Application number: 12797203.2
(22) Date of filing: 06.06.2012
(51) Int. Cl.: C12Q 1/02

(54) **PHOTOTOXICITY TEST METHOD**

(30) Priority: 10.06.2011 JP 2011129912
(71) Applicant: Sumitomo Chemical Company, Limited, Tokyo 104-8260 (JP)
(72) Inventor: ANDO, Satoshi, Osaka-shi Osaka 554-8558 (JP); SAITO, Koichi, Osaka-shi Osaka 554-8558 (JP)
(74) Representative: Vossius & Partner
(86) International application number: PCT/JP2012/065064
(87) International publication number: WO 2012/169658

(57) **Abstract**

The present invention provides a phototoxicity test method using human retinal pigment epithelial cells, and so on.

## Description

### TECHNICAL FIELD

The present invention relates to a phototoxicity test method using human retinal pigment epithelial cells, and so on.

### BACKGROUND ART

In order to evaluate the safety to human of chemical substances such as medicines, agricultural chemicals, cosmetics and industrial products, a number of toxicity tests are usually carried out using animal individuals. Among these toxicity tests, acute toxicity tests for irritability and the like caused by reacting chemical substances having been introduced to a body with light are generally referred to as phototoxicity tests.

As the most widely used method of phototoxicity test methods, for example, the Morikawa method by local administration to animal skin is known. The phototoxicity test method is a test method in which a guinea pig or a white rabbit is used and a chemical substance as a test article is applied to the dorsal skin of the animal, and the determination results obtained by determining a biological reaction in the dorsal skin against UV irradiation to the application site with the naked eye in accordance with an evaluation criterion decided in advance, and the determination results obtained by determining a biological reaction in the dorsal skin against non UV irradiation to the application site with the naked eye in accordance with the evaluation criterion in the same way as above are compared with each other to evaluate the presence or absence of phototoxicity of the chemical substance as a test article or a degree thereof.

Alternative methods of a phototoxicity test method without using a laboratory animal are, however, strongly demanded because of the problems of animal welfare and a tightening of regulations for animal experimentation seen in, e.g., Europe in recent years.

A number of in vitro phototoxicity test methods, meanwhile, have been developed until now. An example of the most expected methods among such phototoxicity test methods can include the neutral red uptake method (3T3 NRU PT) by "Balb/c 3T3 cell" which is a connective tissue cell and a fibroblast. The 3T3 NRU PT has been approved as an alternative method of a phototoxicity test method scientifically established by ECVAM (European Center for the Validation of Alternative Methods) in 1998, and has been adopted as a test method for screening the presence or absence of phototoxicity of a chemical substance since 2000. The test method is presented to OECD and also known as the only test method decided as a guideline for the in vitro phototoxicity test in 2004 (see, e.g., OECD guidelines for the testing of chemicals: test guideline 432 "In vitro 3T3 NRU phototoxicity test" Paris: Frankreich, OECD Publication Office; 2004).

In recent years, however, the evaluation results of the presence or absence of phototoxicity by 3T3 NRU PT and the like are stored in large quantities and it is becoming evident that the results of false positive are contained therein. The method is not always sufficiently acceptable for the prediction of actual safety to human, and the development of a novel in vitro phototoxicity test method has been demanded (see, e.g., Experimental and Toxicologic Pathology, vol.63, p209-214; 2011).

### DISCLOSURE OF THE INVENTION

The present invention provides a phototoxicity test method using human retinal pigment epithelial cells, and so on.

More specifically, the present invention provides:
1. a phototoxicity test method comprising the following steps (1) to (4) (hereinafter, sometimes referred to as the phototoxicity test method of the present invention):
   (1) a first step of exposuring human retinal pigment epithelial cells to a test substance;
   (2) a second step of culturing the human retinal pigment epithelial cells exposed to the test substance in the first step while irradiating with artificial light with a wavelength distribution in a range of 340 nm to 380 nm within 24 hours after the onset of the contact in the first step;
   (3) a third step of collecting the human retinal pigment epithelial cells cultured in the second step and measuring the cytotoxicity or an index value correlated therewith in the collected cultured cells; and
   (4) a fourth step of evaluating the presence or absence or the level of the phototoxic potential of the test substance on the measurement result in the third step and determining the phototoxic potential of the test substance;
2. the phototoxicity test method according to the above 1, wherein the artificial light is generated by a solar simulator comprising ultraviolet light, visible light and infrared light;
3. the phototoxicity test method according to the above 1 or 2, wherein the artificial light irradiation in the second step is irradiation to achieve a light irradiation dose in a range of 3 J/cm² to 30 J/cm² as a measured value at a wavelength of 360 nm;
4. the phototoxicity test method according to any of the above 1 to 3, wherein, in the fourth step, the measurement result in the third step and the measurement result of the cytotoxicity or an index value correlated therewith in collected cultured cells of control human retinal pigment epithelial cells are compared with each other, and the presence or absence or the level of the phototoxic potential of the test substance is evaluated based on differences between the measurement results;
5. the phototoxicity test method according to the above 4, wherein the control comprises a positive control which is human retinal pigment epithelial cells that have been exposed to a known phototoxic substance;
6. the phototoxicity test method according to the above 5, wherein the phototoxic substance is chlorpromazine (CPZ);
7. the phototoxicity test method according to any of the above 1 to 6, wherein the artificial light irradiation in the second step is irradiation with a light irradiation dose that leads to a significant decrease in the number of viable cells among the cells under the light irradiation conditions relative to the number of viable cells among the cells under conditions where the artificial light is blocked, the cell being at least either the human retinal pigment epithelial cells that have been exposed to the test substance or control human retinal pigment epithelial cells;
8. the phototoxicity test method according to any of the above 1 to 7, wherein the artificial light irradiation in the second step is single continuous irradiation or intermittent irradiation consisting of twice or more of repeated irradiation;
9. the phototoxicity test method according to any of the above 1 to 8, wherein the human retinal pigment epithelial cells are retinal pigment epithelial cells derived from stem cells;
10. the phototoxicity test method according to 9 above, wherein the stem cells are embryonic stem cells, induced pluripotent stem cells or neural stem cells;
11. use of chlorpromazine (CPZ) as a positive control for the phototoxicity test method according to any of the above 1 to 10;
12. a positive control reagent used for the phototoxicity test method according to any of the above 1 to 10, the reagent comprising chlorpromazine (CPZ);
13. use of penicillin G, quinine, bithionol or chlorhexidine as a negative control for the phototoxicity test method according to any of the above 1 to 10;
14. a negative control reagent used for the phototoxicity test method according to any of the above 1 to 10, the reagent comprising penicillin G, quinine, bithionol or chlorhexidine;
15. a method for screening a phototoxic substance, comprising:
   determining the phototoxic potential of a test substance by the phototoxicity test method according to any of the above 1 to 10; and
   selecting a substance having intended phototoxic potential is selected (hereinafter, sometimes referred to as the method for screening a phototoxic potential in the present invention); and
16. a method for screening a substance not causing skin irritation with solar simulator, comprising:
   determining the phototoxic potential of a test substance by the phototoxicity test method according to any of the above 1 to 10; and
   selecting a substance not having intended phototoxic potential (hereinafter, sometimes referred to as the method for screening a substance not causing skin irritation with solar simulator in the present invention); and so on.

According to the phototoxicity test method of the present invention, the phototoxic potential of a test substance can be effectively examined. According to the present invention, there can be further provided a phototoxic substance which can be used as a positive control in the test method.

According to the method for screening a phototoxic substance in the present invention, a desired phototoxic substance or a substance not causing skin irritation with solar simulator can be simply screened.

### BRIEF DESCRIPTION OF THE DRAWINGS

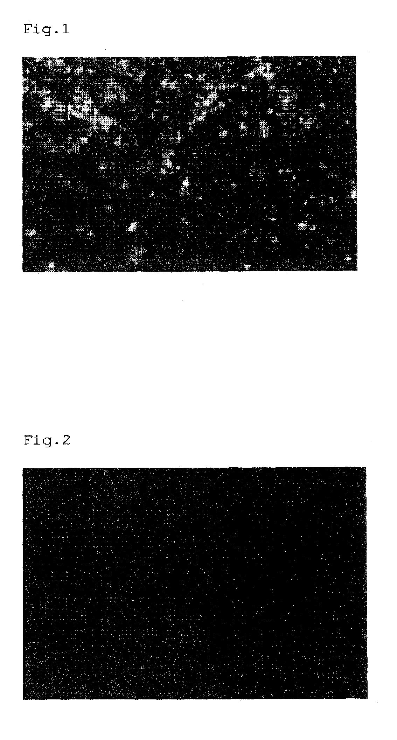

Fig. 1 is a view showing a bright field image of human retinal pigment epithelial cells obtained by differentiation induction from human embryonic stem cells (i.e., ES cells).
Fig. 2 is a view showing the results of immunostain of human retinal pigment epithelial cells (red shows ZO-1, blue shows the nucleus) obtained by differentiation induction from human embryonic stem cells (i.e., ES cells).

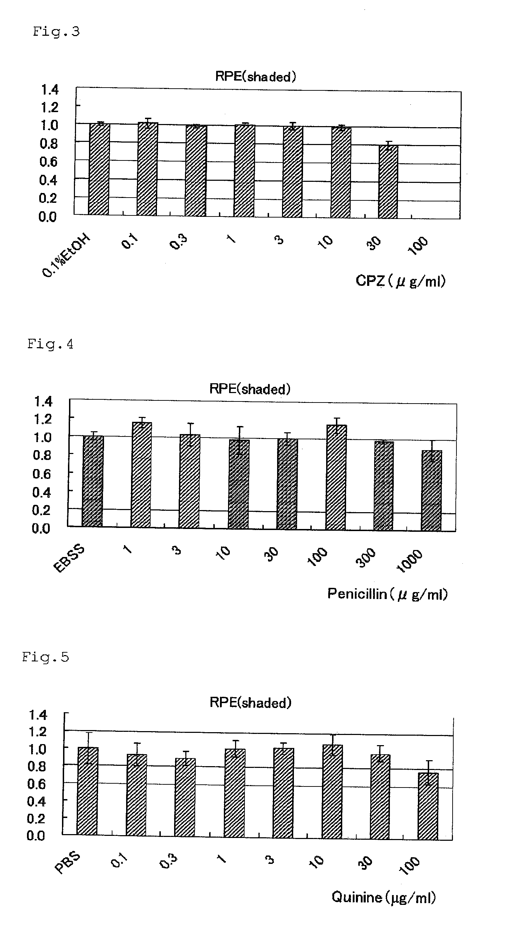

Fig. 3 is a diagram showing the results of the cytotoxicity or an index value correlated therewith measured as a (shaded) control for non-light irradiation by the phototoxicity test method of the present invention using a photocytotoxic compound "chlorpromazine (CPZ)" as a test substance in Example 3.
Fig. 4 is a diagram showing the results of the cytotoxicity or an index value correlated therewith measured as a (shaded) control for non-light irradiation by the phototoxicity test method of the present invention using a photocytotoxicity-negative compound "penicillin G" as a test substance in Example 3.
Fig. 5 is a diagram showing the results of the cytotoxicity or an index value correlated therewith measured as a (shaded) control for non-light irradiation by the phototoxicity test method of the present invention using a photocytotoxicity-negative compound "quinine" as a test substance in Example 3.

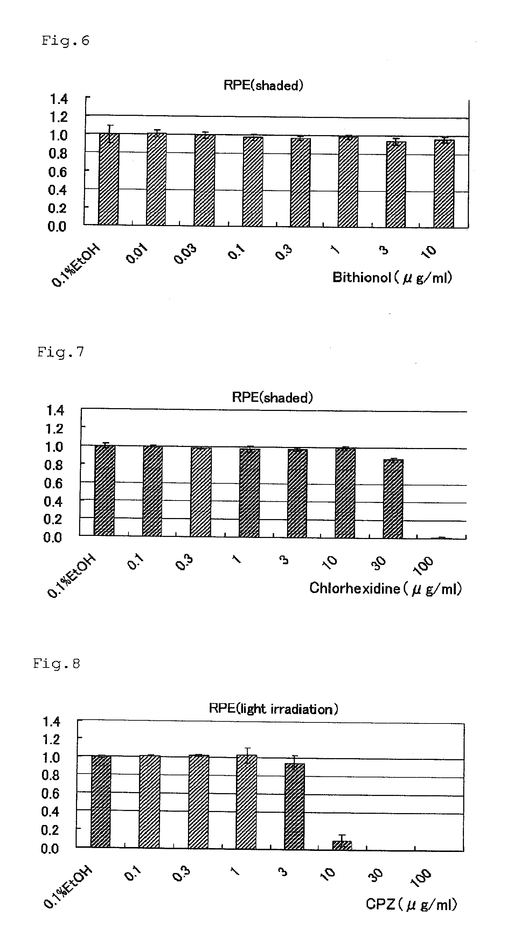

Fig. 6 is a diagram showing the results of the cytotoxicity or an index value correlated therewith measured as a (shaded) control for non-light irradiation by the phototoxicity test method of the present invention using a photocytotoxicity-negative compound "bithionol" as a test substance in Example 3.
Fig. 7 is a diagram showing the results of the cytotoxicity or an index value correlated therewith measured as a (shaded) control for non-light irradiation by the phototoxicity test method of the present invention using a photocytotoxicity-negative compound "chlorhexidine" as a test substance in Example 3.
Fig. 8 is a diagram showing the results of the cytotoxicity or an index value correlated therewith measured under light irradiation conditions by the phototoxicity test method of the present invention using a photocytotoxicity-positive compound "chlorpromazine (CPZ)" as a test substance in Example 4.

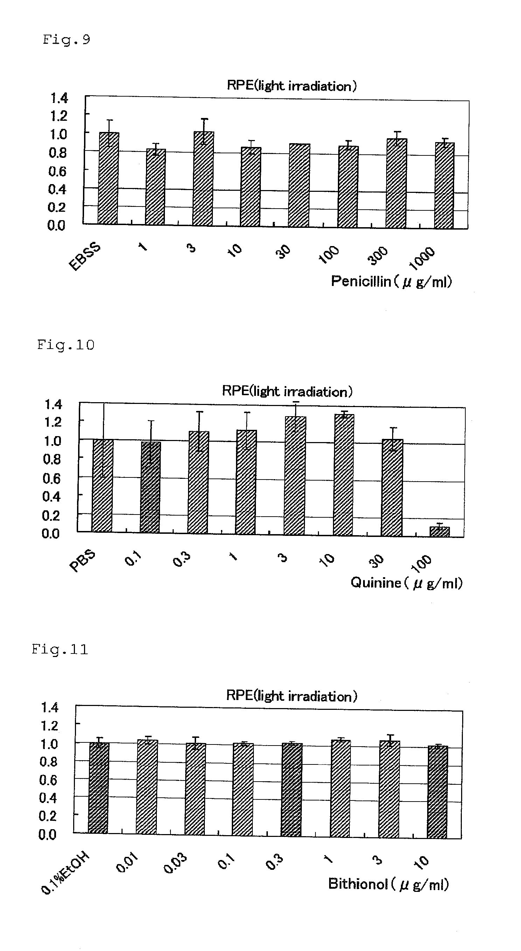

Fig. 9 is a diagram showing the results of the cytotoxicity or an index value correlated therewith measured under light irradiation conditions by the phototoxicity test method of the present invention using a photocytotoxicity-negative compound "penicillin G" as a test substance in Example 4.
Fig. 10 is a diagram showing the results of the cytotoxicity or an index value correlated therewith measured under light irradiation conditions by the phototoxicity test method of the present invention using a photocytotoxicity-negative compound "quinine" as a test substance in Example 4.
Fig. 11 is a diagram showing the results of the cytotoxicity or an index value correlated therewith measured under light irradiation conditions by the phototoxicity test method of the present invention using a photocytotoxicity-negative compound "bithionol" as a test substance in Example 4.

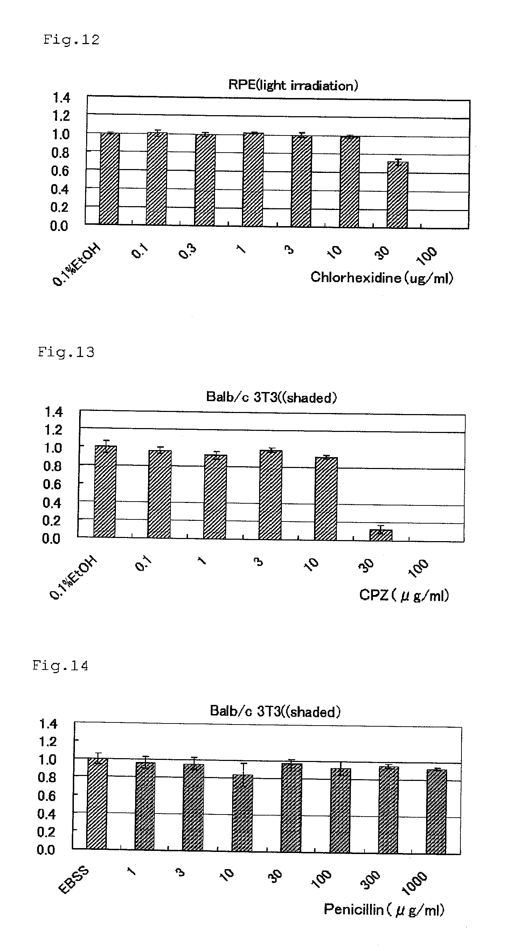

Fig. 12 is a diagram showing the results of the cytotoxicity or an index value correlated therewith measured under light irradiation conditions by the phototoxicity test method of the present invention using a photocytotoxicity-negative compound "chlorhexidine" as a test substance in Example 4.
Fig. 13 is a diagram showing the results of the cytotoxicity or an index value correlated therewith measured as a (shaded) control for non-light irradiation by a comparative phototoxicity test method using a photocytotoxicity-positive compound "chlorpromazine (CPZ)" as a test substance in Comparative Example 1.
Fig. 14 is a diagram showing the results of the cytotoxicity or an index value correlated therewith measured as a (shaded) control for non-light irradiation by a comparative phototoxicity test method using a photocytotoxicity-negative compound "penicillin G" as a test substance in Comparative Example 1.

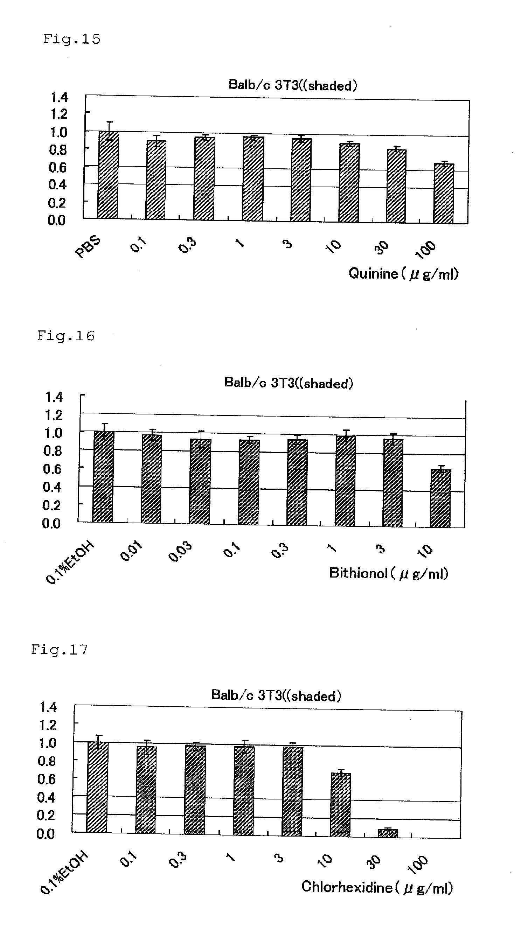

Fig. 15 is a diagram showing the results of the cytotoxicity or an index value correlated therewith measured as a (shaded) control for non-light irradiation by a comparative phototoxicity test method using a photocytotoxicity-negative compound "quinine" as a test substance in Comparative Example 1.
Fig. 16 is a diagram showing the results of the cytotoxicity or an index value correlated therewith measured as a (shaded) control) for non-light irradiation by the phototoxicity test method of the present invention using a photocytotoxicity-negative compound "bithionol" as a test substance in Comparative Example 1.
Fig. 17 is a diagram showing the results of the cytotoxicity or an index value correlated therewith measured by the phototoxicity test method of the present invention (Comparative Example 1: a (shaded) control for non-light irradiation) using a photocytotoxicity-negative compound "chlorhexidine" as a test substance.

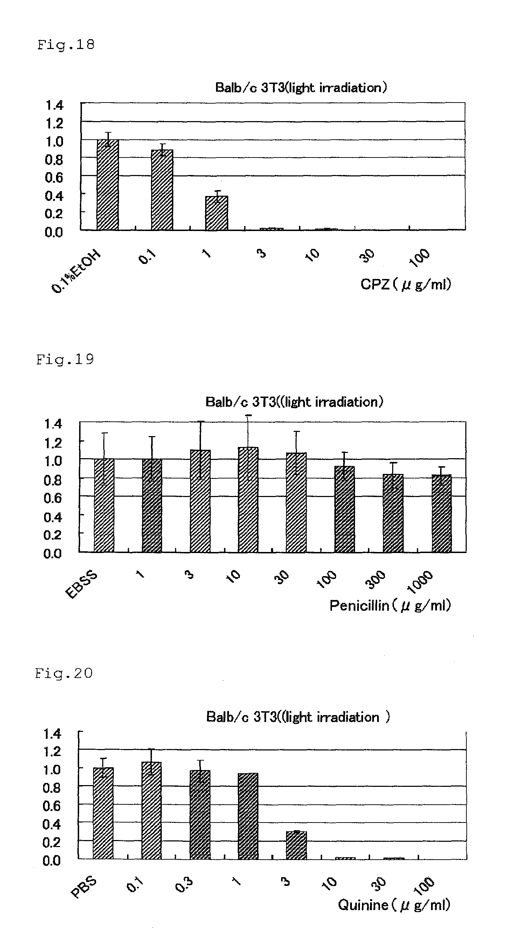

Fig. 18 is a diagram showing the results of the cytotoxicity or an index value correlated therewith measured under light irradiation by a comparative phototoxicity test method using a photocytotoxicity-positive compound "chlorpromazine (CPZ)" as a test substance in Comparative Example 2.
Fig. 19 is a diagram showing the results of the cytotoxicity or an index value correlated therewith measured under light irradiation conditions by a comparative phototoxicity test method using a photocytotoxicity-negative compound "penicillin G" as a test substance in Comparative Example 2.
Fig. 20 is a diagram showing the results of the cytotoxicity or an index value correlated therewith measured under light irradiation conditions by a comparative phototoxicity test method using a photocytotoxicity-negative compound "quinine" as a test substance in Comparative Example 2.

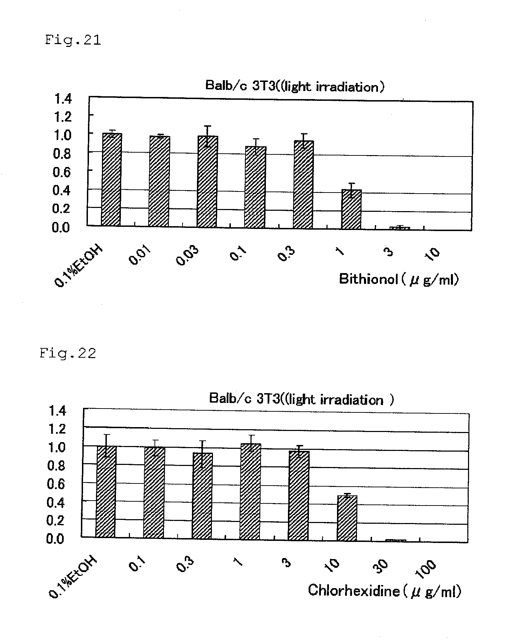

Fig. 21 is a diagram showing the results of the cytotoxicity or an index value correlated therewith measured under light irradiation conditions by a comparative phototoxicity test method using a photocytotoxicity-negative compound "bithionol" as a test substance in Comparative Example 2.
Fig. 22 is a diagram showing the results of the cytotoxicity or an index value correlated therewith measured under light irradiation conditions by a comparative phototoxicity test method using a photocytotoxicity-negative compound "chlorhexidine" as a test substance in Comparative Example 2.

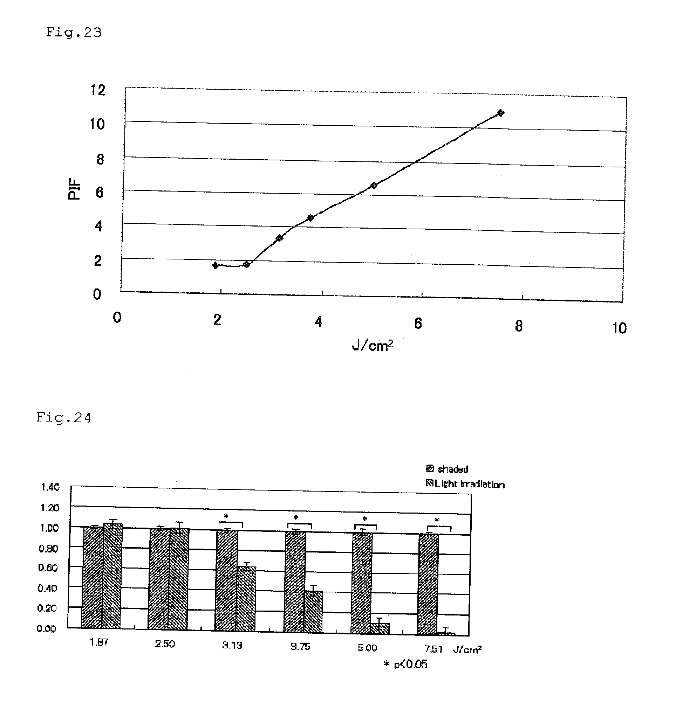

Fig. 23 is a diagram showing the results in a preliminary test (Example 6) for setting light irradiation dose. The diagram shows the examination of the relationship between the light irradiation dose and the above-described PIF value (i.e., a value obtained by dividing the cytotoxicity IC50 value in human retinal pigment epithelial cells exposed to a test substance (a (shaded) control for non-light irradiation) by the cytotoxicity IC50 value in human retinal pigment epithelial cells cultured under light irradiation by a solar simulator after the exposure to the test substance) using chlorpromazine (CPZ) used as a positive control in the OECD guideline.
Fig. 24 is a diagram showing the results in the preliminary test (Example 6) for setting light irradiation dose. The diagram relates to the preliminary test examining the relationship between the light irradiation dose and the above-described PIF value, and confirms that the simulated solar light irradiation is irradiation with a light irradiation dose that leads to a significant decrease in the number of viable cells among the cells under the simulated solar light irradiation conditions relative to the number of viable cells among the cells under conditions where simulated solar light is blocked, the cells being human retinal pigment epithelial cells which have been esposured to 10 µg/ml chlorpromazine (CPZ). The ordinate shows the relative comparative value of the number of viable cells when the number of viable cells under simulated solar light blocked conditions is taken as 1. The bar graph on the left side shows the relative comparative value of the number of viable cells under simulated solar light blocked conditions and the bar graph on the right side shows the relative comparative value of the number of viable cells under light irradiation conditions at each light irradiation dose.

### MODE(S) FOR CARRYING OUT THE INVENTION

Mode(s) for carrying out the present invention will be described in detail below.

A "transformant" in the present invention means the whole or a part of a living organism such as a cell produced by transformation. Examples of the transformants can include procaryotic cells, yeasts, animal cells, plant cells, insect cells and the like. The transformant may be referred to as a transformed cell, a transformed tissue, a transformed host or the like depending on an object thereof. The cell used in the present invention may be a transformant.

Examples of procaryotic cells used for gene manipulation techniques in the present invention can include procaryotic cells belonging to Eschericia genus, Serratia genus, Bacillus genus, Brevibacterium genus, Corynebacterium genus, Microbacterium genus, Pseudomonas genus and the like; more specifically, Eschericia XL1-Blue, Eschericia XL2-Blue, Eschericia DH1 and the like. Such cells are specifically described in, for example, 'Molecular Cloning (3rd edition)' by Sambrook, J and Russell, D.W., Appendix 3 (Volume 3), Vectors and Bacterial strains. A3.2 (Cold Spring Harbor USA 2001).

In relation to the present invention, a "vector" means a vector which can introduce an objective polynucleotide sequence into an objective cell. Examples of such vectors can include those which can be autonomously replicated in procaryotic cells, yeasts, animal cells, plant cells, insect cells and host cells such as animal individuals or plant individuals; those which contain a promoter in a suitable position for transcription of a polynucleotide which can be incorporated into a chromosome; and the like.

Among these vectors, the vectors suitable for cloning can be referred to as "cloning vectors". Such cloning vectors usually comprise a multiple cloning site including a plurality of restriction enzyme sites. Now, a large number of vectors which can be used for gene cloning exist in the art and are sold with different names depending on subtle differences (for example, the kinds and sequences of restriction enzymes in a multi-cloning site) by selling agencies. Typical vectors are described in, for example, 'Molecular Cloning (3rd edition)' by Sambrook, J and Russell, D.W., Appendix 3 (Volume 3), Vectors and Bacterial strains. A3.2 (Cold Spring Harbor USA, 2001)) together with the sellers, and those skilled in the art can properly use these vectors depending on purposes.

In relation to the present invention, the "vector" also includes "expression vectors", "reporter vectors" and "recombinant vectors". The "expression vectors" mean nucleotide sequences in which along with a structural gene and a promoter which regulates the expression thereof, various regulatory elements are linked in an operable state in host cells. Examples of the "regulatory elements" can include those which contain a terminator, a selective marker such as a drug-resistant gene, and an enhancer; and the like. It is a well-known matter for those skilled in the art that types of expression vectors in organisms (e.g., animals) and kinds of regulatory elements to be used can vary depending on host cells.

In relation to the present invention, examples of the "recombinant vectors" can include (a) lambda FIX vector (phage vector) for genome library screening; (b) lambda ZAP vector (phage vector) for cDNA screening; (c) e.g., pBluescript II SK+/-, pGEM and pCR2.1 vectors (plasmid vector) for genome DNA cloning; and the like. Examples of the "expression vectors" can include pSV2/neo vector, pcDNA vector, pUC18 vector, pUC19 vector, pRc/RSV vector, pLenti6/V5-Dest vector, pAd/CMV/V5-DEST vector, pDON-AI-2/neo vector, pMEI-5/neo vector (plasmid vector) and the like. Examples of the "reporter vectors" can include pGL2 vector, pGL3 vector, pGL4.10 vector, pGL4.11 vector, pGL4.12 vector, pGL4.70 vector, pGL4.71 vector, pGL4.72 vector, pSLG vector, pSLO vector, pSLR vector, pEGFP vector, pAcGFP vector, pDsRed vector and the like. Such vectors may be appropriately used by reference to the above-described Molecular Cloning journal.

In relation to the present invention, examples of techniques for introducing nucleic acid molecules into cells can include transformation, transduction, transfection and the like. Specific examples of such introduction techniques can include methods and the like described in, e.g., Ausubel F. A. et al. Ed. (1988), Current Protocols in Molecular Biology, Wiley, New York, NY; Sambrook J. et al. (1987), Molecular Cloning: A Laboratory Manual, 2nd Ed. and the third edition thereof, Cold Spring Harbor Laboratory Press, Cold Spring Harbor, NY; "Experimental Method for Gene Transfer and Expression Analysis" Special Issue of Experimental Medicine, Yodosha (1997) ; and the like. Examples of techniques for confirming the introduction of genes into cells can include northern blot analysis, western blot analysis, other well-known common techniques and the like.

In relation to the present invention, examples of methods for introducing vectors can include transfection, transduction, transformation and the like (e.g., the calcium phosphate method, the liposome method, the DEAE dextran method, the electroporation method, a method using a particle gun (a gene gun), etc.).

The phototoxicity test method of the present invention is a phototoxicity test method comprising the following steps (1) to (4):
(1) a first step of exposuring human retinal pigment epithelial cells to a test substance;
(2) a second step of culturing the human retinal pigment epithelial cells exposed to the test substance in the first step while irradiating with artificial light with a wavelength distribution in a range of 340 nm to 380 nm within 24 hours after the onset of the contact in the first step;
(3) a third step of collecting the human retinal pigment epithelial cells cultured in the second step and measuring the cytotoxicity or an index value correlated therewith in the collected cultured cells; and
(4) a fourth step of evaluating the presence or absence or the level of the phototoxic potential of the test substance on the measurement result in the third step and determining the phototoxic potential of the test substance.

The "test substance" in the present invention is not particularly limited and covers all chemical substances. Examples thereof can include substances used for such purposes as medicines, agricultural chemicals, cosmetics and industrial products; developing substances which have the possibility of being used hereafter; and the like.

Examples of the "phototoxicity" in the present invention can include toxicity caused by toxic reactions involving light irradiation with solar simulator; and the like. Specifically, examples thereof can include photo-irritation as a primary irritant reaction occurring on, e.g., eyes and skin; photo sensitization (photo allergy) which is an allergy reaction; photocarcinogenicity; further photogenotoxicity; and the like.

Examples of the "cytotoxicity" in the present invention can include toxicity affecting the existence of cells such as cell death and inhibition of cell proliferation; and the like. The cytotoxicity or an index value correlated therewith may be measured, for example, by methods usually used such as a method using a hemacytometer, a method by a cell counter, a method using a radioactive isotope such as ³H thymidine (a simple method for measuring only living cells only by incubating a cell culture liquid to which a radioactive isotope such as ³H thymidine is added), the LDH (lactate dehydrogenase) method, a method using neutral red (NR) (a method utilizing properties in which a red pigment, neutral red, is taken into lysosome of living cells and accumulated, which is a simple method for measuring only living cells), a method using crystal violet (CR) (a method utilizing properties in which crystal violet is incorporated into cell membranes of living cells, followed by staining, which is a simple method for measuring only living cells), a method using a tetrazolium salt (a tetrazolium salt is a substrate for intracellular mitochondrial dehydrogenase, and as cells have higher viability, the quantity of tetrazolium salt to be reduced is greater, and the resulting formazan quantity corresponds well to the number of living cells, and thus this is a simple method for measuring only living cells), a method for measuring the expression level of a marker gene (e.g., RPE65, Mitf, ZO1), a method for measuring the ATP level, a method for measuring the melanin level, and a method for measuring the mitochondrial activity by MTT method. When the repeated exposure of a test substance is carried out by repeating "exposure" and "washing and exchanging media" of the test substance and the like, it is preferred that the cytotoxicity in cultured cells collected in each cycle or an index value correlated therewith be measured.

When the cytotoxicity or an index value correlated therewith is measured as described above, generally available measuring kits can be used for the measurement. Examples of kits for measuring the mitochondrial activity, ATP level, lactate dehydrogenase activity and the like can include CellTiter-Glo (registered trademark) Luminescent Cell Viability Assay, Dual-Glo (registered trademark) Luciferase Assay System, Bright-Glo (registered trademark) Luciferase Assay System, Steady-Glo (registered trademark) Luciferase Assay System, Luciferase Assay Systems, ApoTox-Glo (registered trademark) Triplex Assay, CellTiter-Fluor (registered trademark) Cell Viability Assay, CytoTox 96 (registered trademark) Non-Radioactive Cytotoxicity Assay (Promega), ATP Bioluminescence Assay Kit HS II Cell Proliferation Kit I (MTT), Cell Proliferation Kit II (XTT) (Roche), LDH Cytotoxicity Assay Kit (Cayman Chemical), ADP Assay Kit, EnzyLight (BioAssay Systems) and the like.

In the phototoxicity test method of the present invention, a case in which a method for measuring the expression level of a marker gene (e.g., RPE65, Mitf, ZO1) is applied for cytotoxicity or an index value correlated therewith will be described in more detail.

First, the "expression of a marker gene" encompasses the expression of a polynucleotide, oligonucleotide or nucleic acid, or a protein, polypeptide, oligopeptide or peptide, and the like, which are expressed depending on the marker gene.

Examples of the methods for measuring the expression level of a marker gene can include methods for measuring the level of transcription products of mRNA and the level of translation products to polypeptide in, e.g., objective cells; and the like. Examples of methods for measuring the expression level of mRNA (the level of transcription products) can include any suitable methods comprising molecular biological measuring methods such as the northern blot method, the dot blot method, the PCR method, the real time PCR method and a method using a reporter gene. Examples of methods for measuring the expression level of polypeptides (the level of translation products) can include any suitable methods comprising immunological measuring methods such as the ELISA method, the RIA method, the fluorescent antibody method, the western blot method and the immunohistological staining method. The expression variation of a gene means that the expression level at the mRNA stage or the protein stage of a polypeptide evaluated by any suitable methods comprising usual molecular biological measuring methods or immunological measuring methods is increased or decreased. Examples of other methods can include gene analysis methods using an array (e.g., DNA array, protein array) and the like. DNA arrays are described in, for example, Shujunsha Ed. "DNA microarray and the latest PCR method" Special Issue of Cell Technology, and protein arrays are described in Nat Genet. 2002 Dec; 32 Suppl: 526-32 and the like. Examples thereof can further include RT-PCR, the RACE method, the SSCP method, the immunoprecipitation method, the two-hybrid system, in vitro translation and the like. Such methods are described in, for example, Genome Analysis Experimental Method, Yusuke Nakamura's Labo Manual, edited by Yusuke Nakamura, Yodosha (2002) and the like.

Examples of a method utilizing a reporter gene can include a method in which a vector into which a reporter gene operably linked to the promoter sequence of a marker gene is incorporated is produced and this is introduced into human retinal pigment epithelial cells to produce stable transformants; and the like. Because the produced stable transformants express the reporter gene in the cells, when the activity and expression level of the reporter gene are measured, the measured value can be utilized as an index value correlated with the level of viable human retinal pigment epithelial cells.

Examples of the "reporter genes" herein can include firefly luciferase gene (firefly luc), renilla luciferase gene (renilla luc), β-galactosidase gene, chloramphenicol acetyl transferase gene, and a selective marker by which visual selection can be carried out in host cells into which a nucleic acid construct is introduced. Examples of such selective marker genes can include fluorescent pigment marker genes which can be observed in living cells, such as green fluorescent protein (GFP) gene, cyan fluorescent protein (CFP) gene, yellow fluorescent protein (YFP) gene, red fluorescent protein (dsRed) gene and pigment marker genes. It is preferred that the selective marker genes and selective marker proteins do not substantially show toxicity to host cells. Even when the selective marker protein is an enzyme or a fluorescent protein, as a method for detecting the presence or absence of the expression of a reporter gene or a degree thereof, known detection methods and the like may be utilized.

The "promoter" means a region on DNA by which the transcription start site of a gene is determined and frequency thereof is directly adjusted. It is usually a base sequence in which RNA polymerase is bound to start transcription. The "promoter sequence" means a portion having the promoter action of a gene. Usually, the promoter sequence is frequently the first exon of an estimated protein-encoding region or a region within about 5 kbp upstream of the transcription start point, and thus the promoter sequence can be estimated by predicting a protein-encoding region in a genomic base sequence using DNA analysis software. Examples of such DNA analysis software can include DNASIS software (Hitachi Software), GENETYX (GENETYX CORPORATION) and the like. The estimated promoter sequence is different depending on structural genes and usually exists in the upstream of the structural gene, but is not limited thereto and can exist in the downstream of the structural gene.

The sequence to which known transcriptional regulatory factors such as Sp1 and Ap-2 can be bound exists in the region, and the region is expected to play an important action for controlling expression. This supposes that the region comprises at least a base sequence (promoter sequence) which is necessary and sufficient to induce transcription to the region. An accurate position may be checked by using well-known common techniques in the art.

A method for obtaining promoter sequences and predicted DNA sequences from NCBI database will be described.

With respect to a genomic DNA sequence in the upstream of transcription start point predicted to comprise the promoter sequence of human ZO-1 (TJP1) gene, human reference sequence is set as a database to be searched on MCSC Genome Browser homepage on the internet, and by searching "TJP1" as a keyword, the link list of Sequence and Links to Tools and Databases on TJP1 gene will be shown. Move to the "Genomic Sequence Near Gene" page from this link. Next, by checking the check box of Sequence Retrieval Region Options, setting "Promoter/Upstream by" to 5000 bases, further checking the check box of "5' UTR Exons", and then clicking the submit button, the genome DNA sequence of 5' UTR comprising 5kb upstream of the transcription start point and the first exon can be acquired. By searching "TJP1" as a keyword on the NCBI homepage, an accession number NM_003257.3 can be acquired and the mRNA sequence of TJP1 can be obtained. By analyzing these genome sequence and mRNA sequence using DNA analysis software, the DNA sequence in about 5 kbp upstream of the transcription start point can be obtained. As described above, examples of the DNA analysis software can include DNASIS software (Hitachi Software), GENETYX (GENETYX CORPORATION) and the like.

"Being operably linked to a promoter sequence" means arrangement under control of the promoter sequence on the expression (operation) of an intended gene. In order that a promoter is operably linked to a gene, the promoter is usually arranged in the immediate upstream of the gene, but is not necessarily arranged adjacent thereto.

The "human retinal pigment epithelial cells" in the present invention are, for example, cells derived from human and are epithelial cells located in outer layer of the retina. The cells are cells which have an important action for maintaining photoreceptors. The cells are cubic epithelial cells in the form of monolayer, and are mutually bound by tight junction, and are cells having black pigment.

The human retinal pigment epithelial cells are not particularly limited insofar as they can be cultured. In a preferred embodiment, specific examples thereof can include retinal pigment epithelial cells derived from stem cells (i.e., for example, embryonic stem cells (i.e., ES cells), induced pluripotent stem cells (i.e., iPS cells), human retinal pigment epithelial cells which are obtained by differentiation induction from stem cells such as neural stem cells and are easily cultured, etc.); human retinal pigment epithelial cells which are directly acquired from living tissues and are easily cultured; and the like. Examples thereof can further include human retinal pigment epithelial cells derived from cancer cells which are cultured; human retinal pigment epithelial cells which are immortalized by artificial operations and easily cultured; and the like.

The "stem cells" herein mean cells which maintain the same differentiation ability even after cell division, and when a tissue is damaged, the tissue can be reproduced. Examples of the "stem cells" can include embryonic stem cells and tissue stem cells (also referred to as tissue-derived stem cells, tissue-specific stem cells or somatic stem cells); induced pluripotent stem cells (iPS cells) and the like.

Examples of the preferred stem cells used in the phototoxicity test method of the present invention can include embryonic stem cells (i.e., ES cells), induced pluripotent stem cells (i.e., iPS cells), neural stem cells and the like.

The "embryonic stem cells" (i.e., ES cells) are stem cells which have self-renewal ability and multipotency (i.e., pluripotency), and mean pluripotent stem cells derived from early embryos. The embryonic stem cells were first established in 1981 and have been applied to the production of knockout mice since 1989. Human embryonic stem cells were established in 1998 and are being utilized for regenerative medicine. Tissue stem cells comprising neural stem cells and the like are different from embryonic stem cells and are cells whose direction of differentiation is limited, and exist in a specific position in a tissue and have an undifferentiated intracellular structure. Therefore, tissue stem cells have a low degree of pluripotency. Tissue stem cells have a high ratio of nucleus/cytoplasm and are poor in cell organelles. Tissue stem cells generally have multipotency and a slow cell cycle and maintain proliferation potency over a lifetime of an individual. Induced pluripotent stem cells are cells in which multipotency is induced by directly initiating differentiated cells such as fibroblasts by the expression of several kinds of genes such as Oct3/4, Sox2, Klf4 and Myc, and were established using murine cells by Yamanaka et al. in 2006 (Takahashi K, Yamanaka S. Cell. 2006, 126(4), p663-676). The cells were established using human fibroblasts in 2007 and have multipotency as with embryonic stem cells (Takahashi K, Tanabe K, Ohnuki M, Narita M, Ichisaka T, Tomoda K, Yamanaka S. Cell. 2007, 131(5),p861-872., Yu J, Vodyanik MA, Smuga-Otto K, Antosiewicz-Bourget J, Frane JL, Tian S, Nie J, Jonsdottir GA, Ruotti V, Stewart R, Slukvin II, Thomson JA., Science. 2007, 318(5858), p1917-1920., Nakagawa M, Koyanagi M, Tanabe K, Takahashi K, Ichisaka T, Aoi T, Okita K, Mochiduki Y, Takizawa N, Yamanaka S. Nat Biotechnol., 2008, 26(1), p101-106).

The first step in the phototoxicity test method of the present invention is a step of exosuring human retinal pigment epithelial cells to a test substance.

In the first step, human retinal pigment epithelial cells are exposed to a test substance. Examples of the exposure time of the human retinal pigment epithelial cells and the test substance can include one minute or more; preferably 5 minutes or more and within 3 days; more preferably 10 minutes or more and within 24 hours; and the like. Examples of the retention temperature during the exposure can include about 15°C to 42°C, preferably about 35°C to 42°C, more preferably 35°C to 37°C, particularly preferably about 37°C and the like. When the contact is contact in a culture liquid, the pH of the culture liquid can include, for example, about pH 5 to pH 9, and preferably about pH 6.5 to pH 7.8.

Examples of the concentration of carbonic acid gas during the contact of the test substance in the first step can include about 0% to 25% and the like.

Examples of preferred humidity during the contact of the test substance in the first step can include about 95 ± 5% rh, more preferably about 100% rh and the like.

The concentration and amount applied of the test substance which is exposed to the human retinal pigment epithelial cells are not particularly limited, and may be appropriately decided depending on the kinds of test substances, the predicted phototoxic potential of the test substance, the exposure time of the human retinal pigment epithelial cells and the test substance in the first step, the culturing time in the second step, light irradiation dose in the second step and so on. Examples thereof can include about 0.001 µM to 10 mM, preferably 0.01 µM to 5 mM and the like.

Examples of the density of the human retinal pigment epithelial cells in the exposure system of the first step can include about 1 x 10⁴ cells/cm² to 4 x 10⁶ cells/cm², preferably about 4 x 10⁴ cells/cm² to 4 x 10⁵ cells/cm² and the like.

The second step of the phototoxicity test method of the present invention is a step of culturing the human retinal pigment epithelial cells exposed to the test substance in the first step while irradiating with artificial light with a wavelength distribution in a range of 340 nm to 380 nm within 24 hours after the onset of the contact in the first step.

In the second step, the human retinal pigment epithelial cells exposed to the test substance in the first step are cultured while irradiating with artificial light with a wavelength distribution in a range of 340 nm to 380 nm within 24 hours after the onset of the exposure in the first step. When the test substance has phototoxic potential, the phototoxic potential can be expressed in the present step. At this time, it is preferred that a culture liquid not comprising a pigment which absorbs the artificial light and protein components such as serum which can adsorb the test substance be used.

In a preferred embodiment, the cells are cultured while irradiating with artificial light with a wavelength distribution in a range of 340 nm to 380 nm within 12 hours, more preferably within 6 hours, and particularly preferably within about 10 minutes to 2 hours after the onset of the contact in the first step.

In a preferred embodiment in the examination method of the present invention, the first step (i.e., the contact with a test substance) and the second step (i.e., culturing under artificial light irradiation) may be carried out in parallel. More specifically, the second step may be carried out in the state in which the test substance to be exposed in the first step is eliminated, and the second step may be carried out in the state in which the test substance exists.

In the period from the onset of the exposure in the first step to the onset of the artificial light irradiation in the second step, the human retinal pigment epithelial cells exposed to the test substance in the first step, for example, may be cultured under culturing temperature such as about 15°C to 42°C, preferably about 35°C to 42°C, more preferably about 35°C to 37°C, particularly preferably about 37°C and the like under non artificial light irradiation. After the exposure of the human retinal pigment epithelial cells with the test substance within 24 hours in the first step, the cultured cells are washed and the medium is exchanged, followed by carrying out the artificial light irradiation in the second step, thereby the influence of the test substance outside the cells is removed and only the action of the test substance introduced into the cells in the first step can be evaluated.

Examples of the concentration of carbonic acid gas during the cultivation in the second step can include about 0% to 25% and the like.

Examples of humidity during the cultivation in the second step can include about 95 ± 5% rh, preferably about 100% rh and the like.

The artificial light to be irradiated is artificial light with a wavelength distribution in a range of 340 nm to 380 nm. Preferred examples thereof can include artificial light which is simulated solar light comprising ultraviolet light, visual light and infrared light; and the like. More preferred examples thereof can include simulated solar light which does not comprise ultraviolet light with a wavelength of below 305 nm and infrared light with a wavelength of 850 nm or more and comprises light with a wavelength distribution in a range of 315 nm to 800 nm; and the like.

Examples of the artificial light can include light having ultraviolet lamp, mercury lamp, halogen lamp, LED, xenon lamp, metal halide lamp, sodium lamp or the like as a light source; and the like. Preferred examples thereof can include ultraviolet light and simulated solar light having xenon lamp, mercury lamp or the like as a light source; and the like. More preferred examples thereof can include simulated solar light having xenon lamp, mercury lamp or the like as a light source; and the like. After being adjusted so as to have an intended wavelength distribution using a filter which passes only a specific wavelength, a filter which blocks a specific wavelength and the like as needed, the artificial light may be used.

Incidentally, examples of specific light irradiation devices for irradiating simulated solar light can include SOL500, SOL1200, SOL2000 (Dr. K. Honle Medizintechnik GmbH), SUNTEST CPS/CPS+, SUNTEST XLS+ (Atlas Material Testing Technology), SXL-2500V2 and XI-01B140KB1 (SERIC., Ltd.).

With respect to the second step, when the second step is carried out in the state in which a test substance exists, the "exposure time under artificial light irradiation" of the test substance can be adjusted. That is, intended "exposure time under artificial light irradiation" can be achieved by appropriately setting the culturing time and the light irradiation dose of the artificial light. The artificial light irradiation can be continuously or intermittently carried out. When the artificial light is continuously irradiated, the culturing time corresponds to "the contact time under artificial light irradiation". The "contact time under artificial light irradiation" for 15 minutes, 30 minutes, one hour or two hours, for example, can be achieved by culturing the cells for 15 minutes, 30 minutes, one hour or two hours under continuous artificial light irradiation. When the artificial light is intermittently irradiated, meanwhile, the irradiation time of the artificial light corresponds to "the contact time under artificial light irradiation". In the culturing period for 24 hours, for example, the "contact time under artificial light irradiation" for 12 hours can be achieved by culturing the cells under the artificial light irradiation for first 12 hours and then culturing the cells in the state in which the artificial light is blocked for remaining 12 hours.

Examples of the culturing temperature of the human retinal pigment epithelial cells can include about 15°C to 42°C.

In the second step, examples of preferred light irradiation dose when the artificial light with a wavelength distribution in a range of 340 nm to 380 nm is irradiated can include a light irradiation dose in a range of 3 J/cm² to 30 J/cm² as a measured value at a wavelength of 360 nm; and the like. In order to obtain such intended "light irradiation dose", for example, the light irradiation strength of the artificial light, the light irradiation time of the artificial light and the like may be appropriately set. The light irradiation dose (J/cm²) may be calculated, for example, using a numerical formula: time (minute) x light irradiation strength (mW/cm²) x 60/1000. Specifically, for example, it is revealed that by irradiating with artificial light with a light irradiation strength of about 2.08 mW as a measured value at a wavelength of 360 nm for 40 minutes, the light irradiation dose is about 5 J/cm².

In a preferred embodiment, examples of the artificial light irradiation can include irradiation with light irradiation dose that leads to a significant decrease in the number of viable cells among the cells under the light irradiation conditions relative to the number of viable cells among the cells under conditions where the artificial light is blocked, the cells being at least either the human retinal pigment epithelial cells that have been exposed to the test substance or control human retinal pigment epithelial cells. For setting such preferred light irradiation dose, for example, a preliminary test may be carried out according to the method described in Example 6 described below and the like, and preferred light irradiation dose can be easily confirmed.

In a more specific embodiment, examples of the artificial light irradiation can include light irradiation such that it can achieve a light irradiation dose of simulated solar light in a range of 3 J/cm² to 30 J/cm² as a measured value at a wavelength of 360 nm. More preferred examples thereof can include irradiation such that it can achieve a light irradiation dose of simulated solar light in a range of 3.5 J/cm² to 20 J/cm² as a measured value at a wavelength of 360 nm. Particularly preferred examples thereof can include irradiation such that it can achieve a light irradiation dose of simulated solar light in a range of 5 J/cm² to 10 J/cm² as a measured value at a wavelength of 360 nm.

The phototoxic potential of the test substance can be more certainly expressed by irradiating with the above light.

The third step in the phototoxicity test method of the present invention is a step of collecting the human retinal pigment epithelial cells cultured in the second step and measuring the cytotoxicity or an index value correlated therewith in the collected cultured cells.

In the third step, the cytotoxicity or an index value correlated therewith in the collected cultured cells of the human retinal pigment epithelial cells cultured in the second step is measured.

As described above, the cytotoxicity or an index value correlated therewith in the cultured cells may be measured, for example, by methods usually used such as a method using a hemacytometer, a method by a cell counter, a method using a radioactive isotope such as ³H thymidine (a simple method for measuring only living cells only by incubating a cell culture liquid to which a radioactive isotope such as ³H thymidine is added), the LDH (lactate dehydrogenase) method, a method using neutral red (NR) (a method utilizing properties in which a red pigment, neutral red, is taken into lysosome of living cells and accumulated, which is a simple method for measuring only living cells), a method using crystal violet (CR) (a method utilizing properties in which crystal violet is incorporated into cell membranes of living cells, followed by staining, which is a simple method for measuring only living cells), a method using a tetrazolium salt (a tetrazolium salt is a substrate for intracellular mitochondrial dehydrogenase, and as cells have higher viability, the quantity of tetrazolium salt to be reduced is greater, and the resulting formazan quantity corresponds well to the number of living cells, and thus this is a simple method for measuring only living cells), a method for measuring the expression level of a marker gene (e.g., RPE65, Mitf, ZO1), a method for measuring the ATP level, a method for measuring the melanin level, and a method for measuring the mitochondrial activity by MTT method. When the repeated contact of a test substance is carried out by repeating "eposure" and "washing and exchanging media" of the test substance and the like, it is preferred that the cytotoxicity or an index value correlated therewith in cultured cells collected in each cycle be measured.

The fourth step in the phototoxicity test method of the present invention is a step of evaluating the presence or absence or the level of the phototoxic potential of the test substance on the measurement results in the third step and determining the phototoxic potential of the test substance.

In the fourth step, the presence or absence or the level of the phototoxic potential of the test substance is evaluated on the measurement results in the third step and the phototoxic potential of the test substance is determined. When the presence or absence or the level of the phototoxic potential of the test substance is evaluated, the evaluation may be carried out without a control using the absolute values of the measurement results in the third step, or the evaluation may be carried out using controls by comparison with the measurement results.

In a preferred embodiment, the measurement results in the third step and the measurement results of the cytotoxicity or an index value correlated therewith in collected cultured cells of a control human retinal pigment epithelial cell are compared with each other, and the presence or absence or the level of the phototoxic potential of the test substance is evaluated depending on the differences. As the "control" herein, both a negative control and a positive control can be set. Examples of the negative controls include a human retinal pigment epithelial cell group cultured without irradiation of artificial light; a human retinal pigment epithelial cell group cultured under artificial light irradiation with low light irradiation dose; a human retinal pigment epithelial cell group which is exposed to a substance which is previously known not to have phototoxic potential (e.g., solvent only); and the like. When such a negative control is used, examination described below can be carried out. That is, the measurement results of the cytotoxicity or an index value correlated therewith in the collected cultured cells of the test substance and the measurement results of the cytotoxicity or an index value correlated therewith in the collected cultured cells of the negative control are compared with each other. When the cytotoxicity or an index value correlated therewith in the collected cultured cells of the test substance is lower, the test substance can be also evaluated and determined to have phototoxic potential.

In a further preferred embodiment, the control comprises a positive control which is a human retinal pigment epithelial cell that has been exposed to a known phototoxic substance. An example of the positive controls includes a human retinal pigment epithelial cell group which is exposed to a standard substance which is previously known to have phototoxic potential and then cultured under artificial light irradiation. When such a positive control is used, examination described below can be carried out. That is, the measurement results of the cytotoxicity or an index value correlated therewith in the collected cultured cells of the test substance and the measurement results of the cytotoxicity or an index value correlated therewith in the collected cultured cells of the positive control are compared with each other. When the cytotoxicity or an index value correlated therewith in the collected cultured cells of the test substance is lower, the test substance can be also evaluated and determined to have higher phototoxic potential than the standard substance used for the positive control. Further, the phototoxic potential of the test substance can be quantitatively evaluated and determined by evaluating and determining the test substance at each concentration or each amount applied of the standard substance in a similar manner. In this case, the positive control and the negative control may be used in combination. In addition, when the cytotoxicity or an index value correlated therewith in the collected cultured cells of the positive control is lower than that of the negative control, it can also be shown that artificial light is correctly irradiated and the test is valid.

In a further preferred embodiment, chlorpromazine (CPZ) is used for a positive control as a standard substance.

The use of chlorpromazine (CPZ) as a positive control for the phototoxicity test method of the present invention is also one of the present invention. Further, the use of chlorpromazine (CPZ) as a positive control for the phototoxicity test method of the present invention is also one of the present invention.

An aspect of the positive control reagent of the present invention is a positive control reagent used for the phototoxicity test method of the present invention, which contains chlorpromazine (CPZ). As the form of the reagent, chlorpromazine (CPZ) may be directly used or may be dissolved in a solvent to obtain the form of a solution. Further, a form in which chlorpromazine is contained or dispersed in an appropriate base may be used.

In a further preferred embodiment, penicillin G, quinine, bithionol or chlorhexidine is used for a negative control as a standard substance.

The use of penicillin G, quinine, bithionol or chlorhexidine as a negative control for the phototoxicity test method of the present invention is also one of the present invention. Further, the use of penicillin G, quinine, bithionol or chlorhexidine as a negative control for the phototoxicity test method of the present invention is also one of the present invention.

An aspect of the negative control reagent of the present invention is a negative control reagent used for the phototoxicity test method of the present invention, which contains penicillin G, quinine, bithionol or chlorhexidine. As the form of the reagent, penicillin G, quinine, bithionol or chlorhexidine may be directly used or may be dissolved in a solvent to obtain the form of a solution. Further, a form in which penicillin G, quinine, bithionol or chlorhexidine is contained or dispersed in an appropriate base may be used.

Further, the following method can be exemplified as an embodiment with respect to more specific preferred comparison and evaluation. For example, a value (PIF value: Photo Irritation Facor value) is calculated, which is obtained by dividing the cytotoxicity IC50 value in human retinal pigment epithelial cells exposed to a test substance (a (shaded) control for non-light irradiation) by the cytotoxicity IC50 value in human retinal pigment epithelial cells cultured under artificial light irradiation within 24 hours after the onset of contact with the test substance. When the value (PIF value) is greater than 2 and less than 5, the test substance is evaluated as pseudopositive, and when the value is 5 or more, the test substance is evaluated as phototoxicity-positive.

The method for screening a phototoxic potential in the present invention is a method in which the phototoxic potential of a test substance is determined by the phototoxicity test method of the present invention and a substance having intended phototoxic potential is selected. Also in the method for screening a phototoxic substance in the present invention, the same embodiment as in the phototoxicity test method of the present invention can be used. When the negative control is used, for example, the measurement results of the cytotoxicity or an index value correlated therewith in the collected cultured cells of the test substance and the measurement results of the cytotoxicity or an index value correlated therewith in the collected cultured cells of the negative control are compared with each other. A test substance, for which the cytotoxicity or an index value correlated therewith in the collected cultured cells is statistically significantly lower than that of the negative control, may be selected, or a test substance, for which the cytotoxicity or an index value correlated therewith in the collected cultured cells has a value of 50% or less of the negative control, may be selected.

When a positive control such as chlorpromazine (CPZ) is used, meanwhile, the measurement results of the cytotoxicity or an index value correlated therewith in the collected cultured cells of the test substance and the measurement results of the cytotoxicity or an index value correlated therewith in the collected cultured cells of the positive control are compared with each other. A test substance, for which the cytotoxicity or an index value correlated therewith in the collected cultured cells is statistically significantly lower than that of the positive control, is selected, or a test substance, for which when the cytotoxicity or an index value correlated therewith in the collected cultured cells has the same value as of the positive control, the concentration of the test substance is lower than the concentration of the positive control, is selected, thereby being able to select a substance having higher phototoxic potential than a standard substance used for the positive control. When a positive control is used, the cytotoxicity or an index value correlated therewith in the collected cultured cells can be quantitatively evaluated and determined. Specifically, the phototoxic potential of a test substance can be quantitatively evaluated and determined by evaluating and determining the test substance in a similar manner at each concentration or each amount applied of a standard substance such as chlorpromazine (CPZ), and a substance having intended phototoxic potential is selected.

The method for screening a substance not causing skin irritation with simulated solar light in the present invention is a method in which the phototoxic potential of a test substance is determined by the phototoxicity test method of the present invention and a substance not having intended phototoxic potential is selected. Also in the method for screening a substance not causing skin irritation with simulated solar light in the present invention, the same embodiment as in the phototoxicity test method of the present invention can be used. The substances to be screened are not limited insofar as the substances do not have intended phototoxic potential, and examples thereof can include low molecular weight compounds, proteins, peptides and the like.

The phototoxic potential of a test substance can be efficiently examined by a device with a system process based on the phototoxicity test method of the present invention, and a desired phototoxic substance or a substance not causing skin irritation with simulated solar light can be also more simply screened. By recording toxicity information on skin irritation with simulated solar light obtained by the phototoxicity test method of the present invention in an electronic information medium, a useful electronic information medium containing toxicity information can be produced.

### EXAMPLES

The present invention will be described in more detail by way of Examples.

### Example 1

(Differentiation induction of human ES cells into human retinal pigment epithelial cells)

As human embryonic stem cells (i.e., ES cells), KhES-1 strain, a cell strain established by Kyoto University, was used.

The KhES-1 strain was cultured while maintaining an undifferentiated state on mouse embryonic fibroblast (MEF) feeder cells treated with mitomycin C in a medium for cultivation while maintaining human ES cells (containing DMEM/F12, KSR (Knockout serum replacement), nonessential amino acids, L-glutamine, 2-mercaptoethanol, etc.), supplemented with bFGF (basic fibroblast growth factor). After the cultivation, the cells were collected from a culture dish using a dissociation solution with trypsin/collagenase IV to obtain human embryonic stem cell (i.e., ES cell) colonies.

The obtained colonies were broken into a few tens of cells per cell cluster by pipetting using a micropipette, and the colonies with a few hundreds to a few thousands of cell clusters of human embryonic stem cells (i.e., ES cells) were then seeded in 10 ml of medium for cultivation while maintaining human ES cells, supplemented with 1 µ.M to 10 µM SB431542, 1 µM to 10 µM CKI-7 and 1 µM to 10 µM Y27632, in a 10 cm culture dish previously coated with a 0.1% gelatin liquid. This was then cultured in an incubator at 37°C and 5% CO₂.

On the next day (cultivation for one day), half of the medium (5 ml) was removed without taking floated cell clusters, and 5 ml of SDIA medium (containing GMEM, KSR, nonessential amino acids, sodium pyruvate, 2-mercaptoethanol, etc.) supplemented with 1 µM to 10 µM Y27632 was added to the culture dish and cultivation was continued in the same manner as above.

After the cultivation for 5 days, 5 ml of the medium was removed without taking unadhesive cell clusters, and 5 ml of SDIA medium supplemented with 1 µM to 10 µM Y27632 was added to the culture dish and cultivation was further continued in the same manner as above.

After the cultivation for 8 days, the whole medium was removed and 10 ml of PA6 conditioned medium (culture supernatant after PA6 cells in a confluent state were cultured in SDIA medium for one day) was then added to the culture dish. After that, the medium was exchanged using PA6 conditioned medium at every 3 to 4 days. By the operation, differentiation into human retinal pigment epithelial cells having black pigment was induced (see Fig. 1 and Fig. 2).

### Example 2

### (Cultivation of human retinal pigment epithelial cells)

The colonies with human retinal pigment epithelial cells having black pigment obtained by differentiation induction in Example 1 were scratched up using a micropipette and then treated with 0.05% trypsin to disperse the cells.

A medium, in which 2 to 20 ng/ml of bFGF was added to a DMEM/F12 culture liquid supplemented with N2-supplement, was added to a 60 mm culture dish previously coated with Matrigel and the obtained dispersed cells were seeded in the medium. Unspecific cells other than the human retinal pigment epithelial cells were also contained in the dispersed cells seeded as described above.

The cells were cultured in an incubator at 37°C and 5% CO₂ to reach a confluent state and then treated with 0.25% trypsin to collect the cells from the culture dish. The collected cells were seeded in a 10 cm culture dish previously coated with Matrigel and the cells were then cultured in an incubator at 37°C and 5% CO₂ until reaching a confluent state. When the cells reached a confluent state, a great majority of cells existing in the culture dish were human retinal pigment epithelial cells.

The cells were further cultured in a DMEM/F12 culture liquid supplemented with N2-supplement without bFGF for one week or more and then used for the following experiment.

### Example 3

### (First step, Second step and Third step of phototoxicity test method of present invention under shaded conditions as control for non-light irradiation)

The human retinal pigment epithelial cells prepared in Example 2 were treated with 0.25% trypsin to collect the cells from a culture dish. The collected cells were seeded in a 96-well culture dish previously coated with Matrigel at 100000 or 120000 cells per well and then cultured in an incubator at 37°C and 5% CO₂.

After the cultivation for 2 hours to 4 hours, the medium was removed from the 96-well culture dish using an aspirator and Earle's Balanced Salt Solution (EBSS) was then added to the 96-well culture dish at 150 µl per well to wash the cells.

Next, solutions of test substances previously prepared to have the following concentrations using EBSS or a solvent control was added to the culture dish at 100 µl per well.

### <Solutions of test substances>

(a) Chlorpromazine (CPZ) : Concentration 0.1, 1, 3, 10, 30 and 100 (µg/ml)
(b) Penicillin G: Concentration 1, 3, 10, 30, 100, 300 and 1000 (µg/ml)
(c) Quinine: Concentration 0.1, 0.3, 1, 3, 10, 30 and 100 (µg/ml)
(d) Bithionol: Concentration 0.01, 0.03, 0.1, 0.3, 1, 3 and 10 (µg/ml)
(e) Chlorhexidine: Concentration 0.1, 0.3, 1, 3, 10, 30 and 100 (µg/ml)

After the 96-well culture dish was shaded by wrapping with aluminum foil, irradiation of the culture dish shaded with aluminum foil with light was carried out using a light irradiation device (a solar simulator SXL-2500V2: SERIC LTD.) for 40 minutes (light irradiation dose of about 5 J/cm²). Immediately after light irradiation, the 96-well culture dish was washed twice with 150 µl of a DMEM/F12 culture liquid and a DMEM/F12 culture liquid supplemented with N2-supplement was then added to the 96-well culture dish at 100 µl per well, followed by cultivation overnight.

After the cultivation, the medium was removed from the 96-well culture dish and a previously prepared mixed solution of equal amounts of DMEM/F12 culture liquid and Cell titer Glo reaction liquid was then added to the 96-well culture dish at 100 µl per well.

The obtained 96-well culture dish was allowed to stand at room temperature for 30 minutes while shaking in a shaded state with aluminum foil and 95 µl of culture contained in the 96-well culture dish was then transferred to a white 96-well examining dish. Next, the amount of luminescence of the culture contained in the 96-well examining dish (the amount of luminescence has a correlation with the cell survival rate) was measured by EnVision Multilabel Reader (PerkinElmer). The 50% inhibitory concentration (IC50) of a test substance to the human retinal pigment epithelial cells was calculated from the measured values and the calculated value was considered as a value showing cytotoxicity (an index value correlated therewith).

The following results were obtained according to the calculation method.

### <Cytotoxicity of test substances>

(a) Chlorpromazine (CPZ): IC50 = 37.6 (µg/ml) (see Fig. 3)
(b) Penicillin G: IC50 > 1000 (µg/ml) (cytotoxicity was not observed at 1000 µg/ml or less) (see Fig. 4)
(c) Quinine: IC50 = 147.1 (µg/ml) see Fig. 5)
(d) Bithionol: IC50 > 10 (µg/ml) (cytotoxicity was not observed at 10 µg/ml or less) (see Fig. 6)
(e) Chlorhexidine: IC50 = 42.7 (µg/ml) (see Fig. 7)

### Example 4

### (First step, Second step and Third step of phototoxicity test method of present invention)

The human retinal pigment epithelial cells prepared in Example 2 were treated with 0.25% trypsin to collect the cells from a culture dish. The collected cells were seeded in a 96-well culture dish previously coated with Matrigel at 100000 or 120000 cells per well and then cultured in an incubator at 37°C and 5% CO₂.

After the cultivation for 2 hours to 4 hours, the medium was removed from the 96-well culture dish using an aspirator and Earle's Balanced Salt Solution (EBSS) was then added to the 96-well culture dish at 150 µl per well to wash the cells.

Next, solutions of test substances previously prepared to have the following concentrations using EBSS or a solvent control was added to the culture dish at 100 µl per well.

### <Solutions of test substances>

(a) Chlorpromazine (CPZ): Concentration 0.1, 1, 3, 10, 30 and 100 (µg/ml)
(b) Penicillin G: Concentration 1, 3, 10, 30, 100, 300 and 1000 (µg/ml)
(c) Quinine: Concentration 0.1, 0.3, 1, 3, 10, 30 and 100 (µg/ml)
(d) Bithionol: Concentration 0.01, 0.03, 0.1, 0.3, 1, 3 and 10 (µg/ml)
(e) Chlorhexidine: Concentration 0.1, 0.3, 1, 3, 10, 30 and 100 (µg/ml)

Unlike Example 3, irradiation with light was carried out using a light irradiation device (a solar simulator SXL-2500V2: SERIC LTD.) for 40 minutes without wrapping the 96-well culture dish with aluminum foil (light irradiation dose is about 5 J/cm² by calculation based on light irradiation strength at 360 nm). Immediately after light irradiation, the 96-well culture dish was washed twice with 150 µl of a DMEM/F12 culture liquid and a DMEM/F12 culture liquid supplemented with N2-supplement was then added to the 96-well culture dish at 100 µl per well, followed by cultivation overnight.

After the cultivation, the medium was removed from the 96-well culture dish and a previously prepared mixed solution of equal amounts of DMEM/F12 culture liquid and Cell titer Glo reaction liquid was then added to the 96-well culture dish at 100 µl per well.

The obtained 96-well culture dish was allowed to stand at room temperature for 30 minutes while shaking in a shaded state with aluminum foil and 95 µl of culture contained in the 96-well culture dish was then transferred to a white 96-well examining dish. Next, the amount of luminescence of the culture contained in the 96-well examining dish (the amount of luminescence has a correlation with the cell survival rate) was measured by EnVision Multilabel Reader (PerkinElmer). The 50% inhibitory concentration (IC50) of a test substance to the human retinal pigment epithelial cells was calculated from the measured values and the calculated value was considered as a value showing cytotoxicity (an index value correlated therewith).

The following results were obtained according to the calculation method.

### <Cytotoxicity of test substances>

(a) Chlorpromazine (CPZ) : IC50 = 5.8 (µg/ml) (see Fig. 8)
(b) Penicillin G: IC50 > 1000 (µg/ml) (cytotoxicity was not observed at 1000 µg/ml or less) (see Fig. 9)
(c) Quinine: IC50 = 88.9 (µg/ml) (see Fig. 10)
(d) Bithionol: IC50 > 10 (µg/ml) (cytotoxicity was not observed at 10 µg/ml or less) (see Fig. 11)
(e) Chlorhexidine: IC50 = 43.7 (µg/ml) (see Fig. 12)

### Example 5

### (Phototoxicity test method of present invention: Fourth step)

The above-described PIF value (Photo Irritation Facor value) was obtained based on the 50% inhibitory concentration (IC50) calculated in Example 3 and Example 4 (that is, a value obtained by dividing the cytotoxicity IC50 value in human retinal pigment epithelial cells exposed to a test substance (a (shaded) control for non-light irradiation) by the cytotoxicity IC50 value in human retinal pigment epithelial cells cultured under simulated solar light irradiation within 24 hours after the onset of exposure to the test substance). The results are shown as follows. When the value (PIF value) was greater than 5, the test substance was evaluated as phototoxicity-positive.

### <PIF values (Photo Irritation Facor values) of test substances>

### (a) Chlorpromazine (CPZ): 6.5

In the evaluation by the phototoxicity test method of the present invention, chlorpromazine was determined to be photocytotoxicity-positive. This corresponds to the results of previous in vivo tests.

### (b) Penicillin G: Photocytotoxicity is not confirmed.

In the evaluation by the phototoxicity test method of the present invention, penicillin G was determined to be photocytotoxicity-negative. This corresponds to the results of previous in vivo tests.

### (c) Quinine: 1.7

In the evaluation by the phototoxicity test method of the present invention, quinine was determined to be photocytotoxicity-negative. This corresponds to the result of the in vivo test (mouse tail test) described in Ljunggren B. et al., Phototoxic properties of quinine and quinidine: two quinoline methanol isomer.; Photodermatology 5 133-138 (1988).

### (d) Bithionol: Photocytotoxicity is not confirmed.

In the evaluation by the phototoxicity test method of the present invention, bithionol was determined to be photocytotoxicity-negative. This corresponds to originally correct negative results. In the yeast-RBC assay in 4 facilities by Report on Validation Study of Alternatives of Phototoxicity Assays (August 27, 2004), Committee for the Validation Study on Phototoxicity Assays (chairperson: Isao YOSHIMURA), the result was false positive, and the phototoxicity test method of the present invention was confirmed to be excellent.

### (e) Chlorhexidine: 0.98

In the evaluation by the phototoxicity test method of the present invention, chlorhexidine was determined to be photocytotoxicity-negative. This corresponds to originally correct negative results. In the yeast-RBC assay in 4 facilities by Report on Validation Study of Alternatives of Phototoxicity Assays (August 27, 2004), Committee for the Validation Study on Phototoxicity Assays (chairperson: Isao YOSHIMURA), the result was false positive, and the phototoxicity test method of the present invention was confirmed to be excellent.

### Comparative Example 1

### (Comparative experiment using human cells other than human retinal pigment epithelial cells)

"Balb/c 3T3 cells" are connective tissue cells and fibroblasts.

The "Balb/c 3T3 cells" cultured in DMEM medium supplemented with 10% bovine serum and L-glutamine were treated with 0.25% trypsin to collect the cells from a culture dish. The collected cells were seeded in a 96-well culture dish previously coated with a 0.1% gelatin solution at 10000 cells per well and then cultured in DMEM medium supplemented with 10% bovine serum and L-glutamine in an incubator at 37°C and 5% CO₂ overnight.

After the cultivation, the medium was removed from the 96-well culture dish using an aspirator and Earle's Balanced Salt Solution (EBSS) was then added to the 96-well culture dish at 150 µl per well to wash the cells.

Next, solutions of test substances previously prepared to have the following concentrations using EBSS or a solvent control was added to the culture dish at 100 µl per well.

### <Solutions of test substances>

(a) Chlorpromazine (CPZ): Concentration 0.1, 1, 3, 10, 30 and 100 (µg/ml)
(b) Penicillin G: Concentration 1, 3, 10, 30, 100, 300 and 1000 (µg/ml)
(c) Quinine: Concentration 0.1, 0.3, 1, 3, 10, 30 and 100 (µg/ml)
(d) Bithionol: Concentration 0.01, 0.03, 0.1, 0.3, 1, 3 and 10 (µg/ml)
(e) Chlorhexidine: Concentration 0.1, 0.3, 1, 3, 10, 30 and 100 (µg/ml)

After the 96-well culture dish was shaded by wrapping with aluminum foil, irradiation of the culture dish shaded with aluminum foil with light was carried out using a light irradiation device (a solar simulator SXL-2500V2: SERIC LTD.) for 40 minutes (the light irradiation dose is about 5 J/cm² by calculation based on irradiation strength at 360 nm). Immediately after light irradiation, the 96-well culture dish was washed twice with 150 µl of a DMEM/F12 culture liquid and a DMEM/F12 culture liquid supplemented with N2-supplement was then added to the 96-well culture dish at 100 µl per well, followed by cultivation overnight.

After the cultivation, the medium was removed from the 96-well culture dish and a previously prepared mixed solution of equal amounts of DMEM/F12 culture liquid and Cell titer Glo reaction liquid was then added to the 96-well culture dish at 100 µl per well.

The obtained 96-well culture dish was allowed to stand at room temperature for 30 minutes while shaking in a shaded state with aluminum foil and 95 µl of culture contained in the 96-well culture dish was then transferred to a white 96-well examining dish.

Next, the amount of luminescence of the culture contained in the 96-well examining dish (the amount of luminescence has a correlation with the cell survival rate) was measured by EnVision Multilabel Reader (PerkinElmer). The 50% inhibitory concentration (IC50) of a test substance to the human retinal pigment epithelial cells was calculated from the measured values and the calculated value was considered as a value showing cytotoxicity (an index value correlated therewith).

The following results were obtained according to the calculation method.

### <Cytotoxicity of test substances>

(a) Chlorpromazine (CPZ): IC50 = 19.4 (µg/ml) (see Fig. 13)
(b) Penicillin G: IC50 > 1000 (µg/ml) (cytotoxicity was not observed at 1000 µg/ml or less) (see Fig. 14)
(c) Quinine: IC50 = 292.2 (µg/ml) (see Fig. 15)
(d) Bithionol: IC50 = 11.4 (µg/ml) (cytotoxicity was not observed at 3 µg/ml or less) (see Fig. 16)
(e) Chlorhexidine: IC50 = 13.7 (µg/ml) (see Fig. 17) Comparative Example 2

### (Comparative phototoxicity test method: First step, Second step and Third step)

The "Balb/c 3T3 cells" cultured in DMEM medium supplemented with 10% bovine serum and L-glutamine were treated with 0.25% trypsin to collect the cells from a culture dish. The collected cells were seeded in a 96-well culture dish previously coated with a 0.1% gelatin solution at 10000 cells per well and then cultured in DMEM medium supplemented with 10% bovine serum and L-glutamine in an incubator at 37°C and 5% CO₂ overnight.

After the cultivation, the medium was removed from the 96-well culture dish using an aspirator and Earle's Balanced Salt Solution (EBSS) was then added to the 96-well culture dish at 150 µl per well to wash the cells.

Next, solutions of test substances previously prepared to have the following concentrations using EBSS or a solvent control was added to the culture dish at 100 µl per well.

### <Solutions of test substances>

(a)Chlorpromazine (CPZ): Concentration 0.1, 1, 3, 10, 30 and 100 (µg/ml)
(b) Penicillin G: Concentration 1, 3, 10, 30, 100, 300 and 1000 (µg/ml)
(c)Quinine: Concentration 0.1, 0.3, 1, 3, 10, 30 and 100 (µg/ml)
(d) Bithionol: Concentration 0.01, 0.03, 0.1, 0.3, 1, 3 and 10 (µg/ml)
(e)Chlorhexidine: Concentration 0.1, 0.3, 1, 3, 10, 30 and 100 (µg/ml)

Unlike Comparative Example 1, light irradiation was carried out using a light irradiation device (a solar simulator SXL-2500V2: SERIC LTD.) for 40 minutes without wrapping the 96-well culture dish with aluminum foil (light irradiation dose is about 5 J/cm² by calculation based on light irradiation strength at 360 nm). Immediately after light irradiation, the 96-well culture dish was washed twice with 150 µl of a DMEM/F12 culture liquid and a DMEM/F12 culture liquid supplemented with N2-supplement was then added to the 96-well culture dish at 100 µl per well, followed by cultivation overnight.

After the cultivation, the medium was removed from the 96-well culture dish and a previously prepared mixed solution of equal amounts of DMEM/F12 culture liquid and Cell titer Glo reaction liquid was then added to the 96-well culture dish at 100 µl per well.

The obtained 96-well culture dish was allowed to stand at room temperature for 30 minutes while shaking in a shaded state with aluminum foil and 95 µl of culture contained in the 96-well culture dish was then transferred to a white 96-well examining dish. Next, the amount of luminescence of the culture contained in the 96-well examining dish (the amount of luminescence has a correlation with the cell survival rate) was measured by EnVision Multilabel Reader (PerkinElmer). The 50% inhibitory concentration (IC50) of a test substance to the human retinal pigment epithelial cells was calculated from the measured values and the calculated value was considered as a value showing cytotoxicity (an index value correlated therewith).

The following results were obtained according to the calculation method.

### <Cytotoxicity of test substances>

(a) Chlorpromazine (CPZ): IC50 = 0.82 (µg/ml) (see Fig. 18)
(b) Penicillin G: IC50 > 1000 (µg/ml) (cytotoxicity was not observed at 1000 µg/ml or less) (see Fig. 19)
(c) Quinine: IC50 = 2.3 (µg/ml) (µg/ml) (see Fig. 20)
(d) Bithionol): IC50 = 0.93 (µg/ml) (cytotoxicity was not observed at 10 µg/ml or less) (see Fig. 21)
(e) Chlorhexidine: IC50 = 10.1 (µg/ml) (see Fig. 22) Comparative Example 3

### (Comparative phototoxicity test method: Fourth step)

The above-described PIF value (Photo Irritation Facor value) was obtained based on the 50% inhibitory concentration (IC50) calculated in Comparative Example 1 and Comparative Example 2 (that is, a value obtained by dividing the cytotoxicity IC50 value in human retinal pigment epithelial cells exposed to a test substance (a (shaded) control for non-light irradiation) by the cytotoxicity IC50 value in human retinal pigment epithelial cells cultured under simulated solar light irradiation within 24 hours after the onset of contact with the test substance). The results are shown as follows. When the value (PIF value) was greater than 5, a test substance was evaluated as phototoxicity-positive.

### <PIF values (Photo Irritation Facor values) of test substances>

### (a) Chlorpromazine (CPZ): 23.7

In the evaluation by the comparative phototoxicity test method, chlorpromazine was determined to be photocytotoxicity-positive. This corresponds to the results of previous in vivo tests. (b) Penicillin G: Photocytotoxicity is not confirmed.

In the evaluation by the comparative phototoxicity test method, penicillin G was determined to be photocytotoxicity-negative. This corresponds to the results of previous in vivo tests.

### (c) Quinine: 128.8

In the evaluation by the comparative phototoxicity test method, quinine was determined to be photocytotoxicity-positive. This does not correspond to the result of the in vivo test (mouse tail test) described in Ljunggren B. et al., Phototoxic properties of quinine and quinidine: two quinoline methanol isomer.; Photodermatology 5 133-138 (1988). Therefore, the result was false positive by the phototoxicity test method of the present invention.

### (d) Bithionol: 12.4

In the evaluation by the comparative phototoxicity test method, bithionol was determined to be photocytotoxicity-positive. This does not correspond to originally correct negative results. Therefore, the result was false positive by the comparative phototoxicity test method. In the yeast-RBC assay in 4 facilities by Report on Validation Study of Alternatives of Phototoxicity Assays (August 27, 2004), Committee for the Validation Study on Phototoxicity Assays (chairperson: Isao YOSHIMURA), the result was false positive.

### (e) Chlorhexidine: 1.4

In the evaluation by the comparative phototoxicity test method, chlorhexidine was determined to be photocytotoxicity-negative. This corresponds to originally correct negative results. In the yeast-RBC assay in 4 facilities by Report on Validation Study of Alternatives of Phototoxicity Assays (August 27, 2004), Committee for the Validation Study on Phototoxicity Assays (chairperson: Isao YOSHIMURA), the result was false positive.

### Example 6

### (Preliminary test for setting light irradiation dose)

The relationship between the light irradiation dose and the above-described PIF value (that is, a value obtained by dividing the cytotoxicity IC50 value in human retinal pigment epithelial cells exposed to a test substance (a (shaded) control for non-light irradiation) by the cytotoxicity IC50 value in human retinal pigment epithelial cells cultured under simulated solar light irradiation within 24 hours after the onset of contact with the test substance) was examined using chlorpromazine (CPZ) used as a positive control in the OECD guideline. Light irradiation was set to each condition of a light of 2.09 mW/cm² (measured with UVR300 manufactured by TOPCON CORPORATION equipped with photoreceiving part UD360) for 15 minutes (1.87 J/cm²), 20 minutes (2.50 J/cm²), 25 minutes (3.13 J/cm²), 30 minutes (3.75 J/cm²), 40 minutes (5.00 J/cm²) and 60 minutes (7.51 J/cm²), and the phototoxicity test method was carried out according to the methods described in Example 3 and Example 4. The phototoxicity test method in shaded conditions as a control for non-light irradiation was carried out in the same manner as in the phototoxicity test method under light irradiation except that light was blocked.

Consequently, the 50% inhibitory concentration (IC50) of a test substance to human retinal pigment epithelial cells was calculated from the measured values and the PIF values were further obtained based on the calculated values: PIF = 1.70 (for 15 minutes), PIF = 1.72 (for 20 minutes), PIF = 3.35 (for 25 minutes), PIF = 4.57 (for 30 minutes), PIF = 6.53 (for 40 minutes) and PIF = 10.94 (for 60 minutes) (see Fig. 23).

From the above, PIF was not below 5 at a light irradiation dose of 3.75 J/cm² (for 30 minutes) or less and chlorpromazine (CPZ) as a positive control could not be determined to be positive, and thus it was confirmed from the preliminary test that the light irradiation dose was preferably set to a value higher than 3.75 J/cm².

Next, it was confirmed from the preliminary test that simulated solar light irradiation was irradiation with light irradiation dose by which, in human retinal pigment epithelial cells exposed to chlorpromazine (CPZ), the number of viable cells of the cells under light irradiation conditions significantly decreases relative to the number of viable cells of the cells under simulated solar light blocked conditions.

The number of viable cells of the human retinal pigment epithelial cells was checked for 15 minutes (1.87 J/cm²), 20 minutes (2.50 J/cm²), 25 minutes (3.13 J/cm²), 30 minutes (3.75 J/cm²), 40 minutes (5.00 J/cm²) and 60 minutes (7.51 J/cm²). When the results (see Fig. 3) of chlorpromazine (CPZ) at a contact concentration of 10 µg/ml by which a decrease in the number of viable cells of the cells was not observed under shaded conditions with a light irradiation time of 40 minutes were extracted and compared with each other, it could be confirmed that light irradiation dose by which the number of viable cells of the cells under light irradiation conditions significantly decreases relative to the number of viable cells of the cells under simulated solar light blocked conditions was 3.13 J/cm² (for 25 minutes) or more (see Fig. 24).

Therefore, it was confirmed that when the light irradiation dose was 3.13 J/cm² (for 25 minutes) or more, simulated solar light was appropriately irradiated, and the preferred phototoxicity test method of the present invention could be carried out by using the light irradiation dose.

### Example 7

### (Phototoxicity test method of present invention using transformants)

A method for generating human embryonic stem cells (i.e., ES cells) transformed using a vector comprising a reporter gene under the expression control of the promoter of genes (Mitf, RPE65, ZO1, etc.) expressed on human retinal pigment epithelial cells will be specifically described.

### (1) Cloning of gene promoter and production of reporter plasmid

The promoter region of ZO-1 is cloned by a method described below.

The human genomic BAC DNA with 20 ng of genomic DNA extracted from human embryonic stem cells (i.e., ES cells) or RPE65 gene is used as a template, and using primers designed at positions about 5 kb upstream of the translation start point and the transcription start point of RPE65 gene, an intended DNA fragment is amplified by the PCR method using Platinum Taq polymerase (Invitrogen). The PCR reaction is carried out in the conditions of a reaction at 95°C for 5 minutes, 30 cycles of 95°C for 30 seconds, 55°C for 30 seconds and 72°C for 5 minutes, and a reaction of 72°C for 7 minutes by GeneAmp PCR System 9700 (Applied Biosystems).

The obtained PCR product is purified using PCR purification kit (QIAGEN) and the ends of the PCR product are digested by restriction enzymes, followed by agarose gel electrophoresis for purification.

The purified intended DNA fragment is linked to pGL4.17[Luc2/Neo]vector (Promega) dephosphorylated using Alkaline phosphatase (Takara Bio Inc.) using Ligation kit (Takara Bio Inc.). The obtained DNA fragment is transformed to Escherichia coli DH5α competent cell (Takara Bio Inc.) and this is cultured in LB/ampicillin medium at 37°C overnight. After the cultivation, the emergent colonies are cultured in LB/ampicillin liquid medium, and from the obtained Escherichia coli, a plasmid DNA is extracted. The sequence of the insertion fragment in the extracted plasmid DNA is determined and the presence or absence of variation and the like are confirmed.

In order to use for transfection to human embryonic stem cells (i.e., ES cells), each plasmid is extracted again using Qiafilter plasmid extraction kit (QIAGEN). The obtained plasmid DNA, 20 µg, is linearized by treatment with a restriction enzyme, followed by purification to obtain linear DNA.

### (2) Method for generating recombinant human embryonic stem cells (i.e., ES cells)

Human embryonic stem cells (i.e., ES cells) cultured while maintaining an undifferentiated state are treated with a dissociation solution to collect human embryonic stem cell (i.e., ES cell) colonies from a culture dish. The collected colonies are suspended in a medium to maintain human embryonic stem cells (i.e., ES cell) in an undifferentiated state, and this is transferred to a 10 cm culture dish coated with 0.1% gelatin and then allowed to stand in an incubator at 37°C and 5% CO₂ for 2 hours to attach feeder cells onto the dish. The supernatant comprising floating human embryonic stem cell (i.e., ES cell) colonies is transferred to a 15 ml tube and the colonies are settled by centrifugation. The colonies collected as described above are enzymatically treated with a trypsin solution and the human embryonic stem cells (i.e., ES cells) are then dissociated to a single cell by pipetting using a micropipette.

Next, the obtained cells are settled by centrifugation. The cells are suspended in a liquid in which Opti-MEM medium, 2 µg of the linear DNA and 8 µL of FuGENE HD (Promega) are mixed in advance, and the cell suspension is allowed to react at room temperature for 5 minutes and then suspended in a medium to maintain an undifferentiated state, supplemented with 1 µM to 10 µM Y27632.

The obtained cell suspension is seeded on neomycin-resistant feeder cells and cultured in an incubator at 37°C and 5% CO₂ overnight. The drug selective cultivation is carried out by adding 100 µg/mL G418 (Invitrogen) to the medium. After 10 to 15 days from the addition of G418, human embryonic stem cell (i.e., ES cell) colonies formed in a culture dish are isolated under a microscope and these are seeded in a 96-well plate. After the drug selective cultivation is continued, the amplified cells are subcultured after another 10 to 15 days and seeded in a 48-well plate. After the drug selective cultivation is continued, a stably transformed cell strain with drug resistance is acquired.

### (3) Exposure of test substance to human retinal pigment epithelial cells utilizing recombinant human embryonic stem cells (i.e., ES cells) under shaded conditions as control for non-light irradiation and calculation of toxicity value

Differentiation of recombinant human embryonic stem cells (i.e., ES cells) into human retinal pigment epithelial cells is induced according to the method described in Example 1.

Next, the human retinal pigment epithelial cells are cultured according to the method described in Example 2 and a test substance is then exposed thereto according to the method described in Example 3.

On the next day of the exposure, the medium is removed from the 96-well culture dish and a previously prepared mixed solution of equal amounts of DMEM/F12 culture liquid and Steady Glo reaction liquid is then added thereto at 100 µl per well.

This is allowed to stand at room temperature for 30 minutes while shaking in a shaded state with aluminum foil and 95 µl is then transferred to a white 96-well culture dish, and the amount of luminescence thereof is measured by EnVision Multilabel Reader (PerkinElmer). Based on the measured values of the amount of luminescence of a solvent control and a test substance at each concentration used, the 50% inhibitory concentration (IC50) of the test substance to the human retinal pigment epithelial cells is calculated and this is considered as a toxicity value.

### (4) Exposure of test substance to human retinal pigment epithelial cells utilizing recombinant human embryonic stem cells (i.e., ES cells) in light irradiation conditions and calculation of toxicity value

Differentiation of recombinant human embryonic stem cells (i.e., ES cells) into human retinal pigment epithelial cells is induced according to the method described in Example 1.

Next, the human retinal pigment epithelial cells are cultured according to the method described in Example 2 and a test substance is then exposed thereto according to the method described in Example 4.

On the next day of the exposure, the medium is removed from the 96-well culture dish and a previously prepared mixed solution of equal amounts of DMEM/F12 culture liquid and Steady Glo reaction liquid is then added thereto at 100 µl per well.

This is allowed to stand at room temperature for 30 minutes while shaking in a shaded state with aluminum foil and 95 µl is then transferred to a white 96-well culture dish, and the amount of luminescence thereof is measured by EnVision Multilabel Reader (PerkinElmer). Based on the measured values of the amount of luminescence of a solvent control and a test substance at each concentration used, the 50% inhibitory concentration (IC50) of the test substance to the human retinal pigment epithelial cells is calculated and this is considered as a toxicity value.

### (5) Evaluation of phototoxic potential of test substance using human retinal pigment epithelial cells utilizing recombinant human embryonic stem cells (i.e., ES cells)

The above-described PIF value (Photo Irritation Facor value) is obtained based on the 50% inhibitory concentration (IC50) calculated in (4) above (that is, a value obtained by dividing the cytotoxicity IC50 value in human retinal pigment epithelial cells exposed to a test substance (a (shaded) control for non-light irradiation) by the cytotoxicity IC50 value in human retinal pigment epithelial cells cultured under simulated solar light irradiation within 24 hours after the onset of exposure to the test substance). Consequently, when the value (PIF value) is greater than 6, the test substance is evaluated as phototoxicity-positive.

### INDUSTRIAL APPLICABILITY

According to the phototoxicity test method of the present invention, the phototoxic potential of a test substance can be effectively examined. According to the present invention, there can be further provided a phototoxic substance which can be used as a positive control in the test method.

## Claims

1. A phototoxicity test method comprising the following steps (1) to (4):
(1) a first step of exposuring human retinal pigment epithelial cells to a test substance;
(2) a second step of culturing the human retinal pigment epithelial cells exposed to the test substance in the first step while irradiating with artificial light with a wavelength distribution in a range of 340 nm to 380 nm within 24 hours after the onset of the contact in the first step;
(3) a third step of collecting the human retinal pigment epithelial cells cultured in the second step and measuring the cytotoxicity or an index value correlated therewith in the collected cultured cells; and
(4) a fourth step of evaluating the presence or absence or the level of the phototoxic potential of the test substance on the measurement result in the third step and determining the phototoxic potential of the test substance.

2. The phototoxicity test method according to claim 1, wherein the artificial light is generated by a solar simulator comprising ultraviolet light, visible light and infrared light.

3. The phototoxicity test method according to claim 1 or 2, wherein the artificial light irradiation in the second step is irradiation to achieve a light irradiation dose in a range of 3 J/cm² to 30 J/cm² as a measured value at a wavelength of 360 nm.

4. The phototoxicity test method according to any of claims 1 to 3, wherein, in the fourth step, the measurement result in the third step and the measurement result of the cytotoxicity or an index value correlated therewith in collected cultured cells of control human retinal pigment epithelial cells are compared with each other, and the presence or absence or the level of the phototoxic potential of the test substance is evaluated based on differences between the measurement results.

5. The phototoxicity test method according to claim 4, wherein the control comprises a positive control which is human retinal pigment epithelial cells that have been exposed to a known phototoxic substance.

6. The phototoxicity test method according to claim 5, wherein the phototoxic substance is chlorpromazine (CPZ).

7. The phototoxicity test method according to any of claims 1 to 6, wherein the artificial light irradiation in the second step is irradiation with a light irradiation dose that leads to a significant decrease in the number of viable cells among the cells under the light irradiation conditions relative to the number of viable cells among the cells under conditions where the artificial light is blocked, the cell being at least either the human retinal pigment epithelial cells that have been exposed to the test substance or control human retinal pigment epithelial cells.

8. The phototoxicity test method according to any of claims 1 to 7, wherein the artificial light irradiation in the second step is single continuous irradiation or intermittent irradiation consisting of twice or more of repeated irradiation.

9. The phototoxicity test method according to any of claims 1 to 8, wherein the human retinal pigment epithelial cells are retinal pigment epithelial cells derived from stem cells.

10. The phototoxicity test method according to claim 9 above, wherein the stem cells are embryonic stem cells, induced pluripotent stem cells or neural stem cells.

11. Use of chlorpromazine (CPZ) as a positive control for the phototoxicity test method according to any of claims 1 to 10.

12. A positive control reagent used for the phototoxicity test method according to any of claims 1 to 10, the reagent comprising chlorpromazine (CPZ).

13. Use of penicillin G, quinine, bithionol or chlorhexidine as a negative control for the phototoxicity test method according to any of claims 1 to 10.

14. A negative control reagent used for the phototoxicity test method according to any of claims 1 to 10, the reagent comprising penicillin G, quinine, bithionol or chlorhexidine.

15. A method for screening a phototoxic substance, comprising:
determining the phototoxic potential of a test substance by the phototoxicity test method according to any of claims 1 to 10; and
selecting a substance having intended phototoxic potential.

16. A method for screening a substance not causing skin irritation with solar simulator, comprising:
determining the phototoxic potential of a test substance by the phototoxicity test method according to any of claims 1 to 10; and
selecting a substance not having intended phototoxic potential.
